# EUROPEAN PATENT SPECIFICATION

(11) **EP 0 326 240 B1**
(45) Date of publication and mention of the grant of the patent: **20.10.1993**
(21) Application number: 89300164.4
(22) Date of filing: 10.01.1989
(51) Int. Cl.: C07C 237/04, C07C 231/00, C07C 231/02

(54) **2-Amino acetamide derivatives**
2-Aminoacetamid Derivate
Dérivés de 2-aminoacétamide

(30) Priority: 20.01.1988 US 145865; 20.01.1988 US 145866; 20.01.1988 US 145943
(43) Date of publication of application: 02.08.1989
(73) Proprietor: FISONS CORPORATION, Rochester, NY 14603 (US)
(72) Inventor: Griffith, Ronald Conrad, Pittsford New York 14534 (US)
(74) Representative: Wright, Robert Gordon McRae

(56) References cited:
- DE-C- 955 508
- GB-A- 1 420 067
- US-A- 2 449 638
- US-A- 4 073 941
- PRAKTIKA AKADEMICES ATHENON, vol. 50, 1980, pages 383-397, K. ANTONIADOU-VYZA et al, "Synthèse de quelques alpha,beta-diphényléthylamines d'un intérêt pharmacologique probable"
- CHEMICAL ABSTRACTS, vol. 85, no. 1, 5 July 1976, page 462, column 1, abstract no. 5705y, Columbus, Ohio, US; & JP-A-75 130 776
- BEILSTEINS HANDBUCH DER ORGANISCHEN CHEMIE, 4. Auflage, 3. Ergänzungswerk, Springer-Verlag, Berlin, Heidelberg, New-York, 1973

## Description

This invention relates to novel chemical compounds, processes for their preparation, pharmaceutical compositions containing them, and methods of treatment involving their use.

According to the invention, there are provided compounds of general formula I, 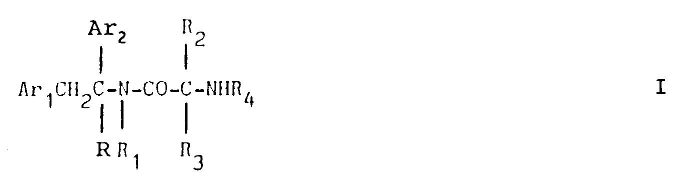
wherein
Ar₁ and Ar₂, which may be the same or different, represent phenyl optionally substituted by fluorine,
R₁ and R₂, which may be the same or different, represent hydrogen or alkyl C₁₋₄,
R₃ represents hydrogen, alkyl C₁₋₄, phenylmethyl or 2-(aminocarbonyl)ethyl, and
R₄ represents hydrogen, alkyl C₁₋₆, cyclopropyl, or a group -COCH(R₅)NH₂ in which R₅ represents hydrogen or methyl,
R represents alkyl C₁₋₄,
provided that, when R₂ and R₃ both represent hydrogen, then R₄ is other than hydrogen,
and pharmaceutically acceptable acid addition salts thereof.

This invention also relates to diastereoisomers and optical isomers (and mixtures thereof) of the compounds of general formula I.

The 2-aminoacetamide derivatives of general formula I as described fully above are conveniently prepared by suitable amide bond forming reactions. Thus, according to a further aspect of the invention, there is provided a process for the preparation of a compound of general formula I, which process comprises
a) producing a compound of general formula I in which R₄ represents hydrogen, alkyl C₁₋₆ or cyclopropyl, by directly coupling an amine of general formula II, 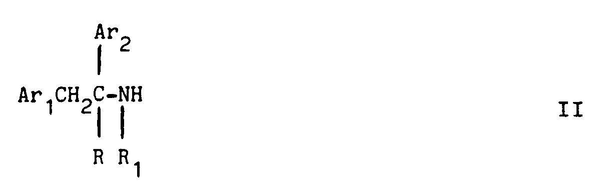 with a compound of general formula III, 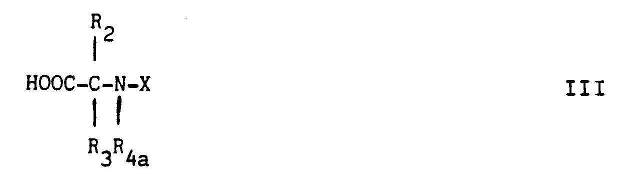 in which R₄ₐ represents hydrogen, alkyl C₁₋₆ or cyclopropyl, and X represents a urethane protecting group, followed by removal of the protecting group X, or
b) producing a compound of general formula I in which R₄ represents hydrogen, alkyl C₁₋₆ or cyclopropyl, by reacting a compound of general formula IV, 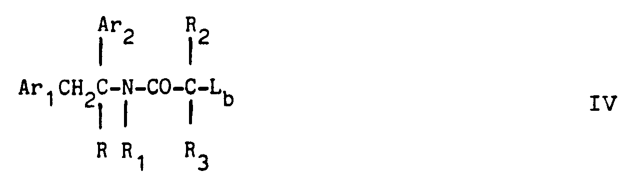 in which L_{b} represents a leaving group, with an amine of general formula V,

   H₂NR_{4b} V

   in which R_{4b} represents hydrogen, alkyl C₁₋₆ or cyclopropyl, or
c) producing a compound of general formula I in which R₄ represents a group -COCHR₅NH₂, by reacting an amine of general formula VI, 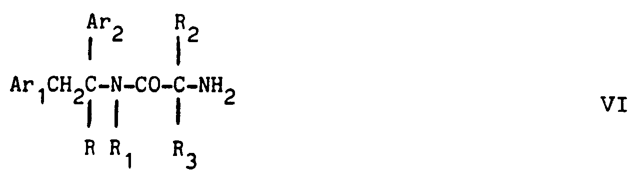 with a compound of general formula VII,

   HO(C=O)CHR₅NHX VII

   in which X is as defined above, followed by removal of the protecting group X,
   and, where necessary or desired, converting the compound of general formula I so obtained to a pharmaceutically acceptable salt thereof or vice versa.

The reaction of process a) is preferably carried out in an inert solvent in the presence of a coupling reagent such as dicyclohexylcarbodiimide with or without 1-hydroxybenzotriazole or other additives. Urethane protecting groups which X may represent include benzyloxycarbonyl (CBZ) and t-butyloxycarbonyl (BOC). The group X may be removed by conventional methods, eg by catalytic hydrogenation in the case of CBZ groups or treatment with an acid such as trifluoroacetic or hydrochloric acid in the case of BOC groups.

Most of the amines of general formula II are known compounds and may be purchased commercially or conveniently prepared by suitable modifications of the reported procedures. Some of the amines II are not known, but are prepared by similar procedures. The preparation of some non-commercially available amines of general formula II is described below (see "Preparation of Intermediates").

The reaction of process b) is preferably carried out in a solvent such a lower alkanol, for example methanol or ethanol, or a chlorinated solvent, for example chloroform or methylene chloride or mixtures thereof. Leaving groups which L_{b} may represent include halide, especially chloride.

Compounds of formula IV may be prepared by reacting an amine of formula II with an activated two carbon acid derivative which contains a leaving group alpha to the carbonyl, ie a compound of general formula VIII, 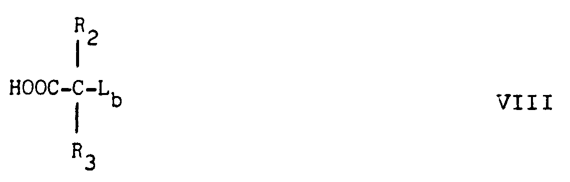
in which L_{b} represents, for example, chloride, in the presence of an acid acceptor, such as triethylamine.

The reaction of process c) may be performed under conditions analogous to those suitable for process a).

The compounds of general formula I possess asymmetric centres, and therefore optical isomers and diastereomeric forms are possible. Such compounds are conveniently prepared from optically active starting materials by the methods described above.

The compounds of general formula I are basic compounds and may be used as such or in the form of their pharmaceutically acceptable acid addition salts. Such salts may be prepared by treatment with various inorganic or organic acids, such as hydrochloric, hydrobromic, sulphuric, phosphoric, acetic, lactic, succinic, furmaric, malic, maleic, tartaric, citric, benzoic, methanesulphonic or carbonic acids.

We prefer compounds in which Ar₁ and Ar₂ are the same, especially compounds in which Ar₁ and Ar₂ represent phenyl.

When either Ar₁ or Ar₂ is substituted by fluorine, it is preferably 4-fluorophenyl.

When any of R, R₁, R₂ and R₃ represent alkyl C₁₋₄, they preferably represent methyl. We prefer compounds in which R₂ represents hydrogen.

As a first specific group of compounds there are provided compounds of general formula I, in which R, R₁ and R₂ are selected from hydrogen and methyl, R₃ represents hydrogen, and R₄ represents -COCHR₅NH₂.

As a second specific group of compounds there are provided compounds of general formula I, in which Ar₁ and Ar₂ are selected from phenyl and 4-fluorophenyl, R₁ represents hydrogen or methyl, R₃ represents alkyl C₁₋₄, phenylmethyl or 2-(aminocarbonyl)ethyl, and R₄ represents hydrogen. A particularly preferred sub-group of such compounds are those in which R represents methyl and Ar₁ and Ar₂ both represent phenyl.

As a third specific group of compounds there are provided compounds of general formula I, in which Ar₁ and Ar₂ are selected from phenyl and 4-fluorophenyl, R₁ and R₂ represent hydrogen or methyl, R₃ represents hydrogen, and R₄ represents alkyl C₁₋₆ or cyclopropyl. A particularly preferred sub-group of such compounds are those in which R represents methyl and Ar₁ and Ar₂ both represent phenyl.

The compounds of general formula I possess useful pharmaceutical properties. In particular they possess useful antiepileptic properties and useful sedative properties. These activities were assessed by standard methods. Antiepileptic activity was measured by assessing a compound's ability to prevent the hind limb tonic extension component of the seizure in groups of mice induced by maximal electroshock (MES) after oral or intraperitoneal administration according to the procedures of the Epilepsy Branch, NINCDS as published by R J Porter et al, Cleve Clin Quarterly (1984) 51, 293, and compared to the standard agents dilantin and phenobarbital. Activities (ED₅₀'s) in the range of 10-400 m/k after oral administration in this assay system were obtained. Sedative activity was assessed by behavioural observation in groups of mice. Selected compounds exhibited activity in the range of 30-600 m/k in this assay.

An important factor in judging the usefulness of antiepileptic agents is an evaluation of their propensity to produce neurotoxic effects (R J Porter, Cleve Clin Quarterly (1984) 51, 293). Selected compounds were evaluated in an acute neurological impairment (NI) assay and NI₅₀ doses determined in mice essentially according to the procedure of Coughenour et al, Pharmac Biochem Behav, (1977) 6, 351. The oral therapeutic index (TI), that is, the NI₅₀ in the neurological impairment assay divided by the ED₅₀ in the maximal electroshock assay after oral doses, was calculated. High oral therapeutic indices were observed.

Thus, according to further aspects of the invention there are provided:
a) a method of treatment of epilepsy, which method comprises administration of a therapeutically effective quantity of a compound of general formula I to a human or animal patient suffering from that condition,
b) the use of a compound of general formula I in the manufacture of a medicament for the treatment of epilepsy,
c) a method of sedation of a human or animal patient, which method comprises administration of a therapeutically effective quantity of a compound of general formula I to that patient, and
d) the use of a compound of general formula I in the manufacture of a medicament for use as a sedative.

Certain 2-amino acetamide derivatives are disclosed as having anti-convulsant activity in DE-C- 955 508.

The dosage administered will naturally depend on the particular compound employed, the mode of administration and the desired effect. However, in general satisfactory results are obtained when the compounds are administered at a dosage of from 0.05µg to 3.5g, which may be administered in divided doses of, for example, 1µg to 750mg.

The compounds of general formula I may be administered by a wide variety of routes and may act systemically or locally. Thus, the compounds may be administered by oral or nasal inhalation to the lung, to the buccal cavity, oesophageally, rectally, topically to the skin or to other available surfaces of the body by injection, eg intravenously, intramuscularly, intraperitoneally, or by surgical implant.

According to the invention there is also provided a pharmaceutical composition comprising preferably less than 80%, and more preferably less than 50%, by weight of a compound of general formula I, or a pharmaceutically acceptable acid addition salt thereof, in admixture with a pharmaceutically acceptable adjuvant, diluent or carrier.

Examples of suitable adjuvants, diluents or carriers are:
for tablets, capsules and dragees - microcrystalline cellulose, calcium phosphate, diatomaceous earth, a sugar such as lactose, dextrose or mannitol, talc, stearic acid, starch, sodium bicarbonate and/or gelatin;
for suppositories - natural or hardened oil or waxes; and for inhalation compositions - coarse lactose.

The invention is illustrated, but in no way limited, by the following examples of the preparation of novel intermediate amines of formula II and novel compounds of general formula I.

### Preparation of Intermediates

### Intermediate A

### 1,2-Diphenyl-2-propylamine hydrochloride.

Prepared by suitable modification of the procedure described by Christol (Bull Soc Chim Fr, 1963, 4, 877) and Ho and Smith (Tetrahedron, 1970, 26, 4277) as follows:

To a suspension of sodium cyanide (34.3 g, 0.7 mol) in 500 ml of glacial acetic acid and 100 ml of n-butylether at 0°C was added portionwise 200 ml of concentrated sulphuric acid. The ice bath was removed and a solution of 1,2-diphenyl-2-propanol (106g, 0.5 mol) in 100 ml of n-butylether was added dropwise over a period of 2 hours, then the mixture was stirred for 48 hours. The mixture was poured into 100 ml of ice, and extracted with chloroform. The extracts were washed with water, dried and evaporated to a solid residue which was stirred with hexane (500 ml), filtered and dried to give 85.35g (72% yield) of N-formyl-1,2-diphenyl-2-propylamine, mp 97-99°C. This was suspended in 1 litre of 10% HCl and heated to reflux for 2.5 hours. After cooling in air for 1 hour then in an ice bath for 30 minutes, the white solid which had crystallised was collected by filtration and vacuum dried to give 85.9g (97% yield) of 1,2-diphenyl-2-propylamine, mp 175-178°C.

### Intermediate B

### 1,2-Bis-(4-fluorophenyl)-2-propylamine hydrochloride.

Prepared by the method of Intermediate A using 1,2-bis(4-fluorophenyl)-2-propanol (prepared by the reaction of 4-fluorobenzyl magnesium chloride and 4′-fluoroacetophenone); mp 188-189°C.

### Intermediate C

### 1,2-Diphenyl-2-butylamine hydrochloride

Prepared by the method of Intermediate A using 1,2-diphenyl-2-butanol (prepared by the reaction of benzylmagnesium chloride and propiophenone); mp 190-192.5°C.

### Intermediate D

### (-)-1,2-Diphenyl-2-propylamine

Racemic 1,2-diphenyl-2-propylamine (86g, 0.4 mol) was dissolved in 0.5 litres 95% ethanol, heated to near reflux and added to a solution of (-)-dibenzoyltartaric acid monohydrate (151.9g, 0.4 mol) in 0.5 litres 95% ethanol also at reflux. A white solid crystallised immediately. The mixture was refluxed for 5 minutes, then allowed to cool to ambient temperature. The solid was collected by filtration and dried to give 86.2g, [α]_{D} = 94.2° (C = 0.5, CH₃OH). The filtrate was saved. The solid was suspended in 0.9 litres of 95% ethanol, stirred and heated to reflux for 1 hour, allowed to cool to ambient temperature and the white solid collected by filtration and vacuum dried at 80°C for 8 hours to give 60.2g of (-)-1,2-diphenyl-2-propylamine (-)-dibenzoyl tartrate, mp 194-195°C, [α]_{D} = -96.0° (C = 0.5, CH₃OH).
5.0g of this salt was dissolved in 250 ml CHCl₃ and 200 ml 5% NH₄OH, shaken vigorously, the layers separated and the organic phase washed with 3 x 200 ml 5% NH₄OH, 2 x 200 ml H₂O and dried over MgSO₄. The solvent was evaporated to give 1.75g of (-)-1,2-diphenyl-2-propylamine as an oil. The maleate salt was prepared by dissolving this oil in 25 ml of ethyl acetate and adding the solution to a hot solution of maleic acid (1.02g, 8.87 mmol) in 50 ml of 3:1 ethyl acetate/isopropanol. Upon cooling a white solid crystallised, which was collected by filtration and vacuum dried to give 2.05g of (-)-1,2-diphenyl-2-propylamine maleate, mp 176-177°C, [α]_{D} = -27.4° (C = 1, CH₃OH).

### Intermediate E

### (+)-1,2-Diphenyl-2-propylamine

The filtrate residue saved in the preparation of Intermediate D was treated with 1 litre CHCl₃ and 0.9 litres of 5% NH₄OH, shaken vigorously, the layers separated and the organic phase washed with 4 x 800 ml 5% NH₄OH and 2 x 500 ml H₂O, then dried over MgSO₄ and evaporated to an oil, which is enriched in (+)-1,2-diphenyl-2-propylamine. This oil (32.3g, 0.153 mol) was dissolved in 200 ml hot 95% ethanol and added to a stirred solution of (+)-dibenzoyl tartaric acid monohydrate (57.55g, 0.153 mol) in 600 ml of refluxing 95% ethanol. A white solid crystallised immediately, which was stirred at reflux for 5 minutes, then allowed to cool to ambient temperature. The solid was collected by filtration and vacuum dried at 80°C for 8 hours to give 71.6g of (+)-1,2-diphenyl-2-propylamine (+)-dibenzoyltartrate, mp 197-198°C, [α]_{D} = +95.8° (C = 0.5, CH₃OH). 5.0g of this salt was dissolved in 250 ml CHCl₃ and 200 ml 5% NH₄OH, shaken vigorously, the layers separated and the organic phase washed with 3 x 200 ml 5% NH₄OH and 2 x 200 ml H₂O and dried over MgSO₄. The solvent was evaporated to give 1.75g of (+)-1,2-diphenyl-2-propylamine as an oil. The maleate salt was prepared by dissolving this oil in 25 ml ethyl acetate and adding the solution to a hot solution of maleic acid (1.02g, 8.78 mmol) in 50 ml 3:1 ethyl acetate/isopropanol. Upon cooling a white solid crystallised, which was collected by filtration and vacuum dried to give 2.06g of (+)-1,2-diphenyl-2-propylamine maleate, mp 177-178°C, [α]_{D} = +27.3° (C = 1, CH₃OH)

### Intermediate F

### N-Methyl-1,2-diphenyl-2-propylamine hydrochloride

N-formyl-1,2-diphenyl-2-propylamine (23.6g, 0.1 mol) was added to a stirred suspension of LiAlH₄ (15.0g, 0.395 mol) in 1 litre of dry tetrahydrofuran. After 2 hours the mixture was heated at 35°C for 22 hours, then refluxed for 2 hours, and allowed to cool to room temperature. Water was added to decompose the excess LiAlH₄, and the mixture filtered to remove the solid salts. Evaporation of the solvent gave 23.0g of the crude product as a yellow oil. This was dissolved in 180 ml of ethyl acetate and 20 ml of isopropanol and acidified with HCl gas. Upon standing a white solid crystallised which was collected by filtration and vacuum dried at 65°C to give 21.7g (84%) of N-methyl-1,2-diphenyl-2-propylamine hydrochloride, mp 200-201°C.

### Intermediate G

### N-Methyl-1,2-diphenylethylamine

To a stirred two phase solution of 1,2-diphenylethylamine (30.0g, 0.15 mol) in 300 ml of methylene chloride and 500 ml of water was added sodium carbonate (23.9g, 0.225 mol) and the solution was cooled to 10°C under nitrogen. Ethyl chloroformate (21.5 ml, 0.225 mol) was added dropwise over a one hour period. The reaction was warmed to ambient temperature and stirred at that temperature for 3 hours. The phases were separated and the aqueous phase was extracted with methylene chloride (75 ml). The combined methylene chloride extracts were washed with 1N HCl (200 ml), brine (200 ml), dried and evaporated to a white solid, 40.3g. Recrystallisation from cyclohexane gave N-carboethoxy-1,2 -diphenylethylamine, mp 74-75°C.

To a stirred suspension of LiAlH₄ (12.4g, 0.032 mol) in 300 ml of tetrahydrofuran at 0°C under nitrogen was added dropwise a solution of N-carboethoxy-1,2-diphenylethylamine (35.0g, 0.13 mmol) in 200 ml of tetrahydrofuran. The mixture was heated to reflux for 8 hours. The mixture was cooled in an ice-water bath and water (13 ml), 15% NaOH (13 ml) and water (39 ml) were carefully added to the mixture. The mixture was warmed to ambient temperature and the precipitated salts were removed by filtration through celite. Removal of solvent gave N-methyl-1,2-diphenylethylamine (26.8g) as a colourless oil.

Treatment of this oil with maleic acid in ethyl acetate and methanol gave N-methyl-1,2-diphenylethylamine maleate, mp 129-131°C.

### Preparation of Compounds of General Formula I

### Example 1

### 2-Glycinamido-N-(1,2-diphenyl-1-methylethyl) acetamide hydrochloride

To a stirred solution of 1,2-diphenyl-2-propylamine (11.5g, 0.055 mol) in 400 ml of chloroform under nitrogen was added N-CBZ-glycylglycine (16.0g, 0.060 mol), and then a solution of dicyclohexylcarbodiimide (11.4g, 0.055 mol) in 125 ml of chloroform and the mixture stirred for 72 hours. The precipated solid was removed by filtration and the solvent evaporated. The residual oil was recrystallised from ethyl acetate (500 ml) and then methanol (500 ml) to give a white solid, 12.9g. This was dissolved in methanol (200 ml) and acidified with HCl gas to give a white solid, 12.5g. This was dissolved in 900 ml of methanol and 90 ml of 10% HCl, and hydrogenated at 40 psi in a Parr apparatus over 3.0g of 10% Pd/C catalyst for 4 hours. The catalyst was removed by filtration, and the solvent evaporated to a white solid (6.5g). This was dissolved in water (500 ml), basified with ice cold NaOH solution, and extracted with ethyl acetate (3 x 250 ml). The combined organic extracts were dried and evaporated to a white solid, 6.3g. This was dissolved in isopropanol (250 ml) and ethyl acetate (150 ml) and acidified with HCl gas. Upon cooling a white solid crystallised, which after vacuum drying at 80°C for 40 hours gave 5.3g of 2-glycinamido-N-(1,2-diphenyl-1-methylethyl) acetamide, mp 226-228°C.

### Example 2

### (2S)-2-Glycinamido-N-(1,2-diphenyl-1-methyl-ethyl) propanamide

Prepared by the method of Example 1 using N-CBZ-glycyl-L-alanine; mp 155-156°C.

### Example 3

### 2-(L-Alaninamido)-N-(1,2-diphenyl-1-methylethyl) acetamide

May be prepared using the method of Example 1 using N-CBZ-L-alanylglycine.

### Example 4

### (2S)-2-(L-alaninamido)-N-(1,2-diphenyl-1-methylethyl) propanamide

Prepared by the method of Example 1 using N-CBZ-L-alanine; mp 161-162°C.

### Example 5

### 2-Glycinoamido-N-[1,2-bis(4-fluorophenyl)-1-methylethyl] acetamide

To a stirred solution of 1,2-bis(4-fluorophenyl)-2-propylamine (8.47g, 0.034 mol) in 200 mol of chloroform under nitrogen, was added N-CBZ-glycylglycine (9.13g, 0.034 mol) and then a solution of dicyclohexylcarbodiimide (7.43g, 0.036 mol) in 100 ml of chloroform and the mixture stirred for 72 hours. The precipitated solid was removed by filtration and the solvent evaporated. The residue was treated with ethylacetate (125 ml) , filtered, an additional 250 ml of ethylacetate added, and the mixture washed with cold 1% HCl (150 ml) , brine (200 ml), dried and the solvent evaporated. The residue was dissolved in 400 ml of methanol and 35 ml of 10% HCl and hydrogenated at 40 psi in a Parr apparatus over 3.0g of 5% Pd/C catalyst for 3 hours. The catalyst was removed by filtration, solvent evaporated and the residue dissolved in water (300 ml) and chloroform (300 ml), basified to pH 11 with 50% NaOH, shaken and separated.

The aqueous phase was extracted with chloroform (3 x 150 ml) , and the combined organic phases were washed with water (2 x 200 ml), dried, and evaporated to a pale yellow oil. This oil was crystallised from cyclohexane (150 ml) and ethanol (5 ml) ; the solid obtained was recrystallised from hexane (150 ml) and ethanol (10 ml) , and vacuum dried at 83°C for 96 hours to give 3.64g of 2-glycinamido-N-[1,2-bis(4-flurophenyl)-1-methylethyl]acetamide,
mp 139-140°C.

### Example 6

By procedures essentially the same as those described in Example 1 using (-) or (+) 1,2-diphenyl-2-propylamine, (-) or (+)-2-glycinamido-N-(1,2-diphenyl-1-methylethyl) acetamide hydrochlorides may be prepared.

### Example 7

By procedures essentially the same as those described in Example 1 using N-methyl-1,2-diphenyl-1-methylethylamine, 2-glycinamido-N-methyl-N-(1,2-diphenyl-1-methylethyl) acetamide hydrochloride may be prepared.

### Example 8

### (2S)-2-Amino-3-phenyl-N-(1,2-diohenyl-1-methylethyl) propanamide hydrochloride

To a stirred solution of 1,2-diphenyl-2-propylamine (10.2g, 0.048 mol) in 400 ml of chloroform under nitrogen were added N-CBZ-L-phenylalanine (14.36g 0.048 mol) and then a solution of dicyclohexylcarbodiimide (9.90g, 0.048 mol) in 130 ml of chloroform and the mixture stirred for 20 hours. The precipitated solid was removed by filtration and the solvent evaporated. Ethyl acetate (200 ml) was added to the residue and the insoluble material was removed by filtration. Ethyl acetate (300 ml) was added to the filtrate. The ethyl acetate solution was washed with 1N HCl (2 x 200 ml), brine (2 x 150 ml) and dried over MgSO₄. Removal of solvent gave 28.72 g of a yellow oil. This oil was dissolved in 350 ml of methanol and 35 ml of 10% HCl, and hydrogenated at 40 psi in a Parr apparatus over 3.0g of 10% Pd/C catalyst for 3.5 hours. The catalyst was removed by filtration and the solvent evaporated. Water (300 ml) was added to the residue, the solution was basified to pH 11 with 50% NaOH, and extracted with chloroform (2 x 200 ml). The combined chloroform extracts were dried over MgSO₄ and the solvent was evaporated to give 21.23g of a yellow oil, This oil was purified by chromatography on silica gel with a Prep 500 HPLC, eluting with ethyl acetate-hexanes (1:1). Pure fractions were combined and evaporated to give 10.5 g of an oil. This oil was dissolved in ethyl acetate (75 ml) and the solution was treated with gaseous HCl. The solvent was removed and the residue was dissolved in methanol and the solvent evaporated (2x). The resulting white solid was triturated with ethyl acetate (75 ml) and recrystallised from ethyl acetate (100 ml) and 2-propanol (0.01 ml) to give 3.7 g of (2S)-2-amino-3-phenyl-N-(1,2-diphenyl-1-methylethyl)propanamide hydrochloride, mp 157-168°C.

### Example 9

### (2R)-2-Amino-3-phenyl-N-(1,2-diphenyl-1-methylethyl)-propanamide hydrochloride

Prepared by the method of Example 8 using N-CBZ-D-phenylalanine; mp 156-157°C.

### Example 10

### (2S)-2-Amino-N-(1,2-diphenyl-1-methylethyl)-propanamide hydrochloride

Prepared by the method of Example 8 using N-CBZ-L-alanine; mp 115-116°C.

### Example 11

### (2R)-2-Amino-N-(1,2-diphenyl-1-methylethyl)-propanamide hydrochloride

Prepared by the method of Example 8 using N-CBZ-D-alanine; mp 115-116°C.

### Example 12

### 2-Amino-N-(1,2-diphenyl-1-methylethyl)-2-methylpropanamide

Prepared by the method of Example 8 using N-CBZ- α,α-dimethylglycine; mp 117-118°C.

### Example 13

### (2S)-2-Amino-4-(aminocarbonyl)-N-(1,2-diphenyl-1-methylethyl)butanamide maleate

Prepared by the method of Example 8 using N-CBZ-L-glutamine; mp 158°C.

### Example 14

### (2R)-2-Amino-4-(aminocarbonyl)-N-(1,2-diphenyl-1-methylethyl)butanamide maleate

Prepared by the method of Example 8 using N-CBZ-D-glutamine; mp 168°C.

### Example 15

### (2S)-2-Amino-N-[1,2-bis(4-fluorophenyl)-1-methylethyl] propanamide

To a stirred solution of 1,2-bis(4-fluorophenyl)-2-propylamine (11.7g, 0.045 mol) in 200 ml of chloroform under nitrogen, were added N-CBZ-L-alanine (10.0g, 0.045 mol) and then a solution of dicyclohexylcarbodiimide (9.90g, 0.05 mol) in 100 ml of chloroform, and the mixture was stirred for 16 hours. The precipitated solid was removed by filtration and the solvent evaporated. The residue was treated with ethyl acetate (100 ml), filtered, an additional 300 ml of ethyl acetate added, and then washed with 1% cold HCl (100 ml), brine (2 x 100 ml), dried over MgSO₄, and the solvent evaporated. The residue was dissolved in 450 ml of methanol and 35 ml of 10% HCl and hydrogenated at 40 psi in a Parr apparatus over 3.0g of 5% Pd/C catalyst for 3 hours. The catalyst was removed by filtration, the solvent evaporated and the residue dissolved in water (200 ml) and chloroform (300 ml), basified to pH 11 with 50% NaOH, shaken and separated. The aqueous phase was extracted with chloroform (2 x 200 ml), and the combined organic phases were washed with water (2 x 200 ml), and dried over MgSO₄. Removal of solvent gave a white solid. This solid was recrystallised from cyclohexane/hexane and then from hexane/ethanol, and vacuum dried to give 1.5g of (2S)-2-amino-N-[1,2-bis(fluorophenyl)-1-methylethyl]-propanamide, mp 134-135°C.

### Example 16

### 2-(Methylamino)-N-(1,2-diphenyl-1-methylethyl) propanamide hydrochloride

Prepared by the method of Example 8 using 1,2-diphenyl-2-propylamine; mp 275-276°C.

### Example 17

### 2-(Methylamino)-N-(1,2-diphenyl-1-methylethyl)-acetamide hydrochloride.

To a stirred solution of 1,2-diphenyl-2-propylamine hydrochloride (77g, 0.31 mol) in 820 ml of chloroform at 0°, was added triethylamine (131g, 1.3 mol) and then chloroacetyl chloride (36.9g, 0.326 mol) and the mixture stirred under nitrogen for 24 hours. 10% HCl (1 litre) was added, stirred for 1 hour then the layers separated. The chloroform layer was washed with 10% HCl (2 x 500 ml), water (2 x 500 ml), dried, and evaporated to a tan solid, 92.0g. This was slurried in 700 ml of hot cyclohexane, filtered, then slurried in 500 ml of hexane, filtered and dried to give 56g of the chloroacetamide as a tan solid, mp 161-162°C. The chloroacetamide (10g, 0.035 mol) was added portionwise to a stirred solution of monomethylamine (25 ml) in 250 ml of methanol 0°C, the mixture was treated with 25 ml chloroform and allowed to warm to room temperature and stirred for 23 hours. All solids were not dissolved so 25 ml of monomethylamine and 50 ml chloroform were added and the mixture stirred for 85 hours, then evaporated to a brown residue. This was dissolved in water (150 ml) and chloroform (100 ml), basified to pH 11 with 50% NaOH, shaken, the layers separated, the aqueous phase extracted with chloroform (2 x 200 ml) and the combined organic phases washed with water (250 ml), dried, and evaporated to a brown residue. This was treated with 250 ml of hexane, stirred vigorously, and the hexane-soluble materials decanted from some dark brown residue. The hexane was evaporated and the oily residue dissolved in ethyl acetate (49 ml) and isopropanol (10 ml), cooled to 0°C and acidified with HCl gas. The solid salt crystallised and was recrystallised twice from 100 ml of isopropanol and 25 ml of ethyl acetate, and dried to give 6.0g of 2-(methylamino)-N-(1,2-diphenyl-1-methylethyl)acetamide hydrochloride, mp 225-226°C.

### Example 18

### 2-(Ethylamino)-N-(1,2-diphenyl-1-methtlethyl)-acetamide hydrochloride

Prepared by the method of Example 17 using ethylamine; mp 164-165°C.

### Example 19

### 2-(propylamino)-N-(1,2-diphenyl-1-methylethyl)-acetamide hydrochloride

Prepared by the method of Example 17 using n-propylamine; mp 188-189°C

### Example 20

### 2-(Isopropylamino-N-(1,2-diphenyl-1-methylethyl)-acetamide hydrochloride

Prepared by the method of Example 17 using isopropylamine for monomethylamine; mp 191°C.

### Example 21

### 2-(Cyclopropylamino)-N-(1,2-diphenyl-1-methylethyl acetamide hydrochloride

Prepared by the method of Example 17 using cyclopropylamine for monomethylamine, mp 194-195°C.

### Example 22

### 2-(Butylamino)-N-(1,2-diphenyl-1-methylethyl)-acetamide hydrochloride

Prepared by the method of Example 17 using n-butylamine; mp 192°C.

### Example 23

### 2-(Hexylamino)-N-(1,2-diphenyl-1-methylethyl)-acetamide hydrochloride

Prepared by the method of Example 17 using n-hexylamine; mp 201-210°C.

### Example 24

### 2-(Methylamino)-N-(1,2-diphenyl-1-methylethyl)acetamide hydrochloride

Prepared by the method of Example 17 using N-methyl-1,2-diphenyl-2-propylamine hydrochloride; mp 205°C.

### Example 25

### (+)-2-(Methylamino)-N-(1,2-diphenyl-1-methylethyl)-acetamide hydrochloride

Prepared by the method of Example 17 using (+)-1,2-diphenyl-2-propylamine hydrochloride; mp 170.5-172.5°C.

### Example 26

### (-)-2-(Methylamino)-N-(1,2-diphenyl-1-methylethyl)-acetamide

Prepared by the method of Example 17 using (-)-1,2-diphenyl-2-propylamine; mp 171-172.5°C.

### Example 27

### 2-(Methylamino)-N-[1,2-bis(4-fluorophenyl)-1-methylethyl]acetamide hydrochloride

To a stirred solution of 1,2-bis(4-fluorophenyl)-2-propylamine (11.08g, 0.045 mol) and triethylamine (9.11g, 0.09 mol) in 130 ml of chloroform under nitrogen at 0°C was added dropwide a solution of chloroacetyl chloride (5.31g, 0.047 mol) in 15 ml of chloroform. The ice bath was removed and the mixture stirred for 19 hours, then poured into 200 ml of 10% HCl. The layers were separated, the organic layer washed with 10% HCl (2 x 80 ml), water (2 x 70 ml), dried, and evaporated to a brown residue. This was dissolved in 250 ml cyclohexane and drying gave 3.57g of the chloroacetamide, mp 156-157°C. 10.0g of material obtained as above (0.031 mol) was added portionwise to a stirred solution of monomethylamine (35 ml) in 250 ml of methanol at 0°C. The ice bath was removed and the mixture stirred for 48 hours. The solvent was evaporated and the residue dissolved in chloroform (350 ml) and water (200 ml) and basified to pH 11 with 50% NaOH. Layers were separated, the aqueous layer extracted with chloroform (2 x 200 ml), and the combined organic layers were washed with water (2 x 500 ml) , dried and evaporated to a brown oil. This was dissolved in 175 ml of ethyl acetate, acidified with HCl saturated isopropanol, and the crystallised solid collected by filtration. Recrystallisation from 175 ml of ethyl acetate, 15 ml of methanol and 10 ml of ethanol, and vacuum drying gave 4.54g of 2-(methylamino)-N-[1,2-bis(4-fluorophenyl)-1-methylethyl]acetamide hydrochloride, mp 250-251°C.

### Example 28

### 2-(Butylamino)-N-[1,2-bis(4-fluorophenyl)-1-methylethyl]acetamide hydrochloride

Prepared by the method of Example 27 using n-butylamine; mp 131-132°C.

### Example 29

### 2-(Methylamino)-N-(1-ethyl-1,2-diphenylethyl)-acetamide hydrochloride

Prepared by the method of Example 17 using 1,2-diphenyl-2-butylamine hydrochloride; 230-231.5°C.

### Example 30

### 2-(Butylamino)-N-(1-ethyl-1,2-diphenylethyl)-acetamide hydrochloride

Prepared by the method of Example 17 using 1,2-diphenyl-2-butylamine hydrochloride and n-butylamine; mp 202-205°C.

## Claims (Claims for the following Contracting State(s): AT, BE, CH, DE, FR, GB, IT, LI, LU, NL, SE)

1. A compound of general formula I, 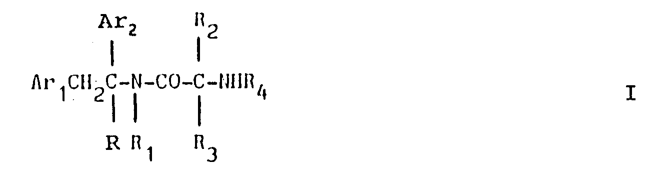 wherein
Ar₁ and Ar₂, which may be the same or different, represent phenyl optionally substituted by fluorine,
R₁ and R₂, which may be the same or different, represent hydrogen or alkyl C₁₋₄,
R₃ represents hydrogen, alkyl C₁₋₄, phenylmethyl or 2-(aminocarbonyl)ethyl, and
R₄ represents hydrogen, alkyl C₁₋₆, cyclopropyl, or a group -COCH(R₅)NH₂ in which R₅ represents hydrogen or methyl,
R represents alkyl C₁₋₄,
provided that, when R₂ and R₃ both represent hydrogen, then R₄ is other than hydrogen,
or a pharmaceutically acceptable salt thereof.

2. A compound according to Claim 1 in which R₂ is hydrogen.

3. A compound according to Claim 1, in which R₁ and R₂ are selected from hydrogen and methyl, R represents methyl, R₃ represents hydrogen and R₄ represents -COCHR₅NH₂.

4. A compound according to Claims 1 or 2, in which Ar₁ and Ar₂ are selected from phenyl and 4-fluorophenyl, R₁ represents hydrogen or methyl, R₃ represents alkyl C₁₋₄, phenylmethyl or 2-(aminocarbonyl)ethyl, and R₄ represents hydrogen.

5. A compound according to Claim 1, in which Ar₁ and Ar₂ are selected from phenyl and 4-fluorophenyl, R₁ and R₂ represent hydrogen or methyl, R₃ represents hydrogen and R₄ represents alkyl C₁₋₄ or cyclopropyl.

6. A compound according to Claim 1 which is
2-Glycinamido-N-(1,2-diphenyl-1-methylethyl acetamide hydrochloride;
(2S)-2-Glycinamido-N-(1,2-diphenyl-1-methylethyl) propanamide;
2-(L-Alaninamido)-N-(1,2-diphenyl-1-methylethyl acetamide;
(2S)-2-(L-alaninamido)-N-(1,2-diphenyl-1-methylethyl) propanamide;
2-Glycinamido-N-[1,2-bis(4-fluorophenyl)-1-methylethyl] acetamide;
(2S)-2-Amino-3-phenyl-N-(1,2-diphenyl-1-methylethyl) propanamide hydrochloride;
(2R)-2-Amino-3-phenyl-N-(1,2-diphenyl-1-methylethyl) propanamide hydrochloride;
(2S)-2-Amino-N-(1,2-diphenyl-1-methylethyl)-propanamide hydrochloride;
(2R)-2-Amino-N-(1,2-diphenyl-1-methylethyl)-propanamide hydrochloride;
2-Amino-N-(1,2-diphenyl-1-methylethyl)-2-methylpropanamide;
(2S)-2-Amino-4-(aminocarbonyl)-N-(1,2-diphenyl-1-methylethyl)butanamide maleate;
(2R)-2-Amino-4-(aminocarbonyl)-N-(1,2-diphenyl-1-methylethyl)butanamide maleate;
(2S)-2-Amino-N-[1,2-bis(4-fluorophenyl)-1-methylethyl] propanamide;
2-(methylamino)-N-(1,2-diphenyl-1-methylethyl) propanamide hydrochloride;
2-(Methylamino)-N-(1,2-diphenyl-1-methylethyl)-acetamide hydrochloride;
2-(Ethylamino)-N-(1,2-diphenyl-1-methylethyl)-acetamide hydrochloride;
2-(propylamino)-N-(1,2-diphenyl-1-methylethyl)-acetamide hydrochloride;
2-(Isopropylamino-N-(1,2-diphenyl-1-methylethyl)-acetamide hydrochloride;
2-(Cyclopropylamino)-N-(1,2-diphenyl-1-methylethyl)-acetamide hydrochloride;
2-(Butylamino)-N-(1,2-diphenyl-1-methylethyl)-acetamide hydrochloride;
2-(Hexylamino)-N-(1,2-diphenyl-1-methylethyl)-acetamide hydrochloride;
2-(Methylamino)-N-(1,2-diphenyl-1-methylethyl)acetamide hydrochloride;
(+)-2-(Methylamino)-N-(1,2-dipnenyl-1-methylethyl) acetamide hydrochloride;
(-)-2-(Methylamino)-N-(1,2-diphrnyl-1-methylethy]) acetamide;
2-(Methylamino)-N-[1,2-bis(4-fluorophenyl)-1-methylethyl]acetamide hydrochloride;
2-(Butylamino)-N-[1,2-bis(4-fluorophenyl)-1-methylethyl]acetamide hydrochloride;
2-(Methylamino)-N-(1-ethyl-1,2-diphenylethyl)-acetamide hydrochloride;
2-(Butylamino)-N-(1-ethyl-1,2-diphenylethyl)-acetamide hydrochloride;
or a pharmaceutically acceptable acid addition salt of any one thereof.

7. The use of a compound of formula I, as defined in Claim 1, in the manufacture of a medicament for the treatment of epilepsy.

8. A pharmaceutical composition comprising less than 80% by weight of a compound of general formula I, or a pharmaceutically acceptable acid addition salt thereof, in admixture with a pharmaceutically acceptable adjuvant, diluent or carrier.

9. A process for the preparation of a compound of general formula I, as defined in Claim 1, which process comprises
a) producing a compound of general formula I in which R₄ represents hydrogen, alkyl C₁₋₆ or cyclopropyl, by directly coupling an amine of general formula II, 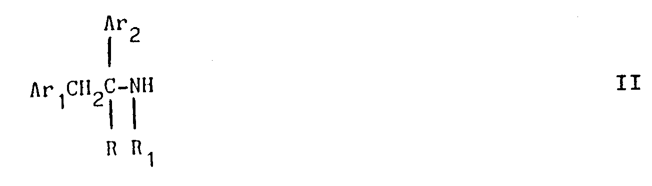 in which R, R₁, Ar₁ and Ar₂ are as defined in Claim 1, with a compound of general formula III, 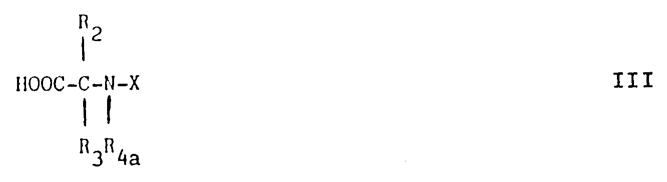 in which R₂ and R₃ are as defined in claim 1, R₄ₐ represents hydrogen, alkyl c₁₋₆ or cyclopropyl, and X represents a urethane protecting group, followed by removal of the protecting group X, or
b) producing a compound of general formula I in which R₄ represents hydrogen, alkyl C₁₋₆ or cyclopropyl, by reacting a compound of general formula IV, 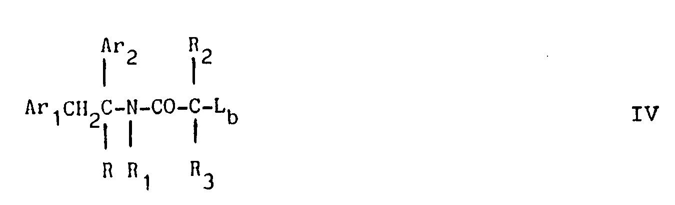 in which R, R₁, R₂, R₃, Ar₁ and Ar₂ are as defined in Claim 1, and L_{b} represents a leaving group, with an amine of general formula V,
H₂NR_{4b} V
in which R_{4b} represents hydrogen, alkyl C₁₋₆ or cyclopropyl, or
c) producing a compound of general formula I in which R₄ represents a group -COCHR₅NH₂, by reacting an amine of general formula VI, 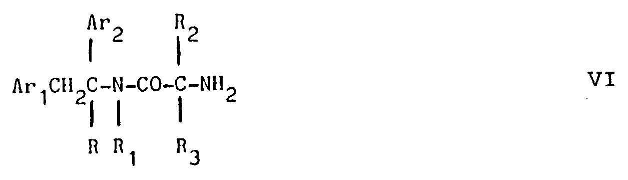 in which R, R₁, R₂, R₃, Ar₁ and Ar₂ are as defined in Claim 1, with a compound of general formula VII,
HO(C=O)CHR₅NHX VII
in which R₅ is as defined in Claim 1,
and X is as defined above, followed by removal of the protecting group X,
and, where necessary or desired, converting the compound of general formula I so obtained to a pharmaceutically acceptable salt thereof or vice versa.

## Claims (Claims for the following Contracting State(s): ES, GR)

1. A process for the preparation of a compound of general formula I, 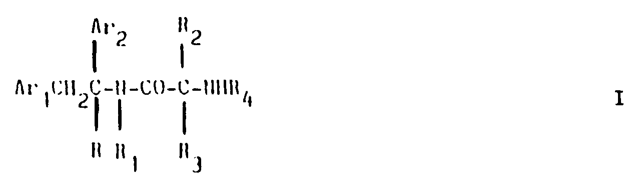 wherein
Ar₁ and Ar₂, which may be the same or different, represent phenyl optionally substituted by fluorine,
R₁ and R₂, which may be the same or different, represent hydrogen or alkyl C₁₋₄,
R₃ represents hydrogen, alkyl c₁₋₄, phenylmethyl or 2-(aminocarbonyl)ethyl, and
R₄ represents hydrogen, alkyl C₁₋₆, cyclopropyl, or a group -COCH(R₅)NH₂ in which R₅ represents hydrogen or methyl,
R represents alkyl C₁₋₄,
provided that, when R₂ and R₃ both represent hydrogen, then R₄ is other than hydrogen,
or a pharmaceutically acceptable acid addition salt thereof, which process comprises
a) producing a compound of general formula I in which R₄ represents hydrogen, alkyl C₁₋₆ or cyclopropyl, by directly coupling an amine of general formula II, 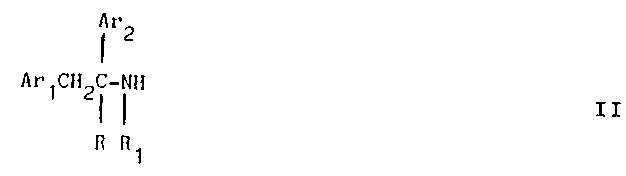 in which R, R₁, Ar₁ and Ar₂ are as defined above, with a compound of general formula III, 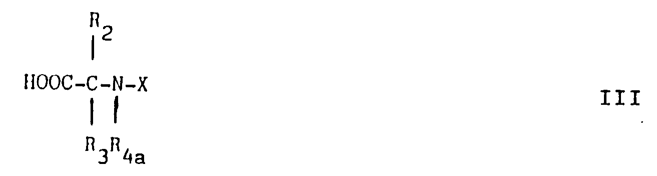 in which R₂ and R₃ are as defined above, R₄ₐ represents hydrogen, alkyl C₁₋₆ or cyclopropyl, and X represents a urethane protecting group, followed by removal of the protecting group X, or
b) producing a compound of general formula I in which R₄ represents hydrogen, alkyl C₁₋₆ or cyclopropyl, by reacting a compound of general formula IV, 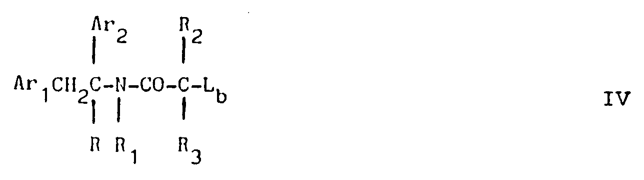 in which R, R₁, R₂, R₃, Ar₁ and Ar₂ are as defined above, and L_{b} represents a leaving group, with an amine of general formula V,
H₂NR_{4b} V
in which R_{4b} represents hydrogen, alkyl C₁₋₆ or cyclopropyl, or
c) producing a compound of general formula I in which R₄ represents a group -COCHR₅NH₂, by reacting an amine of general formula VI, 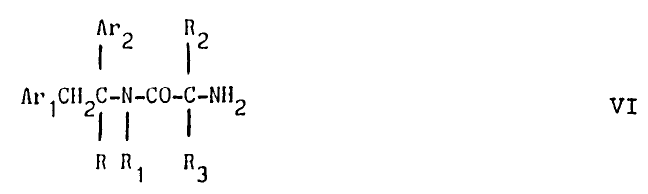 in which R, R₁, R₂, R₃, Ar₁ and Ar₂ are as defined above, with a compound of general formula VII,
HO(C=O)CHR₅NHX VII
in which
R₅ and X are as defined above, followed by removal of the protecting group X,
and, where necessary or desired, converting the compound of general formula I so obtained to a pharmaceutically acceptable salt thereof or vice versa.

2. A process according to Claim 1 in which R₂ is hydrogen.

3. A process according to Claim 1, in which R₁ and R₂ are selected from hydrogen and methyl, R represents methyl, R₃ represents hydrogen and R₄ represents -COCHR₅NH₂.

4. A process according to Claims 1 or 2, in which Ar₁ and Ar₂ are selected front phenyl and 4-fluorophenyl, R₁ represents hydrogen or methyl, R₃ represents alkyl C₁₋₄, phenylmethyl or 2-(aminocarbonyl)ethyl, and R₄ represents hydrogen.

5. A process according to Claim 1, in which Ar₁ and Ar₂ are selected from phenyl and 4-fluorophenyl, R₁ and R₂ represent hydrogen or methyl, R₃ represents hydrogen and R₄ represents alkyl C₁₋₄ or cyclopropyl.

6. A process according to Claim 1, in which the compound of general formula I is:
2-Glycinamido-N-(1,2-diphenyl-1-methylethyl acetamide hydrochloride;
(2S)-2-Glycinamido-N-(1,2-diphenyl-1-methylethyl) propanainide;
2-(L-Alaninamido)-N-(1,2-diphenyl-1-methylethyl) acetamide;
(2S)-2-(L-alaninamido)-N-(1,2-diphenyl-1-methylethyl) propanamide;
2-Glycinamido-N-[1,2-bis(4-fluorophenyl)-1-methylethyl] acetamide;
(2S)-2-Amino-3-phenyl-N-(1,2-diphenyl-1-methylethyl) propanamide hydrochloride;
(2R)-2-Amino-3-phenyl-N-(1,2-diphenyl-1-methylethyl) propanamide hydrochloride;
(2S)-2-Amino-N-(1,2-diphenyl-1-methylethyl)-propanamide hydrochloride;
(2R)-2-Amino-N-(1,2-diphenyl-1-methylethyl)-propanamide hydrochloride;
2-Amino-N-(1,2-diphenyl-1-methylethyl)-2-methylpropanamide;
(2S)-2-Amino-4-(aminocarbonyl)-N-(1,2-diphenyl-1-methylethyl)butanamide maleate;
(2R)-2-Amino-4-(aminocarbonyl)-N-(1,2-diphenyl-1-methylethyl)butanamide maleate;
(2S)-2-Amino-N-[1,2-bis(4-fluorophenyl-1-methylethyl) propanamide;
2-(methylamino)-N-(1,2-diphenyl-1-methylethyl) propanamide hydrochloride;
2-(Methylamino)-N-(1,2-diphenyl-1-methylethyl)-acetamide hydrochloride;
2-(Ethylamino)-N-(1,2-diphenyl-1-methylethyl)-acetamide hydrochloride;
2-(propylamino)-N-(1,2-diphenyl-1-methylethyl)-acetamide hydrochloride;
2-(Isoproptlamino-N-(1,2-diphenyl-1-methylethyl)-acetamide hydrochloride;
2-(Cyclopropylamino)-N-(1,2-diphenyl-1-methylethyl)-acetamide hydrochloride;
2-(Butylamino)-N-(1,2-diphenyl-1-methylethyl)-acetamide hydrochloride;
2-(Hexylamino)-N-(1,2-diphenyl-1-methylethyl)-acetamide hydrochloride;
2-(Methylamino)-N-(1,2-diphenyl-1-methylethyl)acetamide hydrochloride;
(+)-2-(Methylamino)-N-(1,2-diphenyl-1-methylethyl) acetamide hydrochloride;
(-)-2-(Methylamino)-N-(1,2-diphenyl-1-methylethyl) acetamide;
2-(Methylamino)-N-[1,2-bis(4-fluorophenyl)-1-methylethyl]acetamide hydrochloride;
2-(Butylamino)-N-[1,2-bis(4-fluorophenyl)-1-methylethyl]acetamide hydrochloride;
2-(Methylamino)-N-(1-ethyl-1,2-diphenylethyl)-acetamide hydrochloride;
2-(Butylamino)-N-(1-ethyl-1,2-diphenylethyl)-acetamide hydrochloride;
or a pharmaceutically acceptable acid addition salt of any one thereof.

## Patentansprüche (Patentansprüche für folgende(n) Vertragsstaat(en): AT, BE, CH, DE, FR, GB, IT, LI, LU, NL, SE)

1. Verbindung der allgemeinen Formel (I) 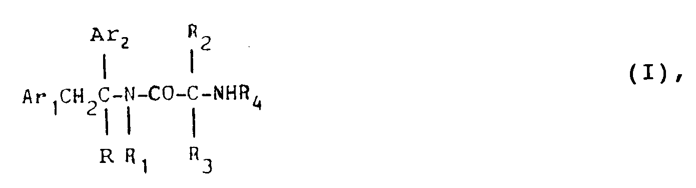 worin Ar₁ und Ar₂, die gleich oder verschieden sein können, gegebenenfalls durch Fluor substituiertes Phenyl darstellen, R₁ und R₂, die gleich oder verschieden sein können, Wasserstoff oder C₁-C₄-Alkyl bedeuten, R₃ Wasserstoff, C₁-C₄-Alkyl, Phenylmethyl oder 2-(Aminocarbonyl)-ethyl ist, und R₄ Wasserstoff, C₁-C₆-Alkyl, Cyclopropyl oder eine Gruppe -COCH(R₅)NH₂ darstellt, worin R₅ Wasserstoff oder Methyl bedeutet, R C₁-C₄-Alkyl ist, vorausgesetzt, daß, wenn R₂ und R₃ beide Wasserstoff darstellen, R₄ von Wasserstoff verschieden ist; oder ein pharmazeutisch annehmbares Salz hievon.

2. Verbindung nach Anspruch 1, worin R₂ Wasserstoff ist.

3. Verbindung nach Anspruch 1, worin R₁ und R₂ aus Wasserstoff und Methyl ausgewählt sind, R Methyl darstellt, R₃ Wasserstoff ist und R₄ die Bedeutung -COCHR₅NH₂ hat.

4. Verbindung nach Anspruch 1 oder 2, worin Ar₁ und Ar₂ aus Phenyl und 4-Fluorphenyl ausgewählt sind, R₁ Wasserstoff oder Methyl darstellt, R₃ C₁-C₄-Alkyl, Phenylmethyl oder 2-(Aminocarbonyl)-ethyl bedeutet und R₄ Wasserstoff ist.

5. Verbindung nach Anspruch 1, worin Ar₁ und Ar₂ aus Phenyl und 4-Fluorphenyl ausgewählt sind, R₁ und R₂ Wasserstoff oder Methyl darstellen, R₃ Wasserstoff bedeutet und R₄ C₁-C₄-Alkyl oder Cyclopropyl ist.

6. Verbindung nach Anspruch 1, welche
2-Glycinamido-N-(1,2-diphenyl-1-methylethyl)-acetamidhydrochlorid;
(2S)-Glycinamido-N-(1,2-diphenyl-1-methylethyl)-propanamid;
2-(L-Alaninamido)-N-(1,2-diphenyl-1-methylethyl)-acetamid;
(2S)-2-(L-Alaninamido)-N-(1,2-diphenyl-1-methylethyl)-propanamid;
2-Glycinamido-N-[1,2,-bis-)4-fluorphenyl)-1-methylethyl]-acetamid;
(2S)-2-Amino-3-phenyl-N-(1,2-diphenyl-1-methylethyl)-propanamidhydrochlorid;
(2R)-2-Amino-3-phenyl-N-(1,2-diphenyl-1-methylethyl)-propanamidhydrochlorid;
(2S)-2-Amino-N-(1,2-diphenyl-1-methylethyl)-propanamidhydrochlorid;
(2R)-2-Amino-N-(1,2-diphenyl-1-methylethyl)-propanamidhydrochlorid;
2-Amino-N-(1,2-diphenyl-1-methylethyl)-2-methylpropanamid;
(2S)-2-Amino-4-(aminocarbonyl)-N-(1,2-diphenyl-1-methylethyl)-butanamidmaleat;
(2R)-2-Amino-4-(aminocarbonyl)-N-(1,2-diphenyl-1-methylethyl)-butanamidmaleat;
(2S)-2-Amino-N-[1,2-bis-(4-fluorphenyl)-1-methylethyl]-propanamid;
2-(Methylamino)-N-(1,2-diphenyl-1-methylethyl)-propanamidhydrochlorid;
2-(Methylamino)-N-(1,2-diphenyl-1-methylethyl)-acetamidhydrochlorid;
2-(Ethylamino)-N-(1,2-diphenyl-1-methylethyl)-acetamidhydrochlorid;
2-(Propylamino)-N-(1,2-diphenyl-1-methylethyl)-acetamidhydrochlorid;
2-(Isopropylamino)-N-(1,2-diphenyl-1-methylethyl)-acetamidhydrochlorid;
2-(Cyclopropylamino)-N-(1,2-diphenyl-]-methylethyl)-acetamidhydrochlorid;
2-(Butylamino)-N-(1,2-diphenyl-1-methylethyl)-acetamidhydrochlorid;
2-(Hexylamino)-N-(1,2-diphenyl-1-methylethyl)-acetamidhydrochlorid;
2-(Methylamino)-N-(1,2-diphenyl-1-methylethyl)-acetamidhydrochlorid;
(+)-2-(Methylamino)-N-(1,2-diphenyl-1-methylethyl)-acetamidhydrochlorid;
(-)-2-(Methylamino)-N-(1,2-diphenyl-1-methylethyl)-acetamid;
2-(Methylamino)-N-[1,2-bis-(4-fluorphenyl)-1-methylethyl]-acetamidhydrochlorid;
2-(Butylamino)-N-[1,2-bis-(4-fluorphenyl)-1-methylethyl]-acetamidhydrochlorid;
2-(Methylamino)-N-(1-ethyl-1,2-dipenylethyl)-acetamidhydrochlorid;
2-(Butylamino)-N-(1-ethyl-1,2-diphenylethyl)-acetamidhydrochlorid;
oder ein pharmazeutisch annehmbares Salz von einem hievon ist.

7. Verwendung einer Verbindung der Formel (I), wie in Anspruch 1 definiert, bei der Herstellung eines Medikamentes zur Behandlung von Epilepsie.

8. Pharmazeutische Zusammensetzung, umfassend weniger als 80 Gew.% einer Verbindung der allgemeinen Formel (I), oder ein pharmazeutisch annehmbares Säureadditionssalz hievon, in Mischung mit einem pharmazeutisch annehmbaren Adjuvans, Verdünnungsmittel oder Träger.

9. Verfahren zur Herstellung einer Verbindung der allgemeinen Formel (I), wie in Anspruch 1 definiert, welches Verfahren:
a) das Herstellen einer Verbindung der allgemeinen Formel (I), worin R₄ Wasserstoff, C₁-C₆-Alkyl oder Cyclopropyl darstellt, durch direktes Koppeln eines Amins der allgemeinen Formel (II) 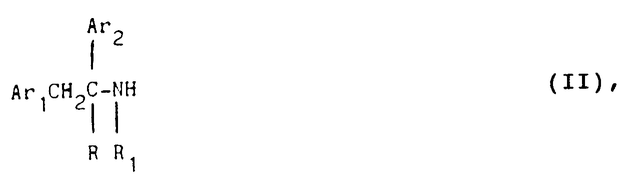 worin R, R₁, Ar₁ und Ar₂ wie in Anspruch 1 definiert sind, mit einer Verbindung der allgemeinen Formel (III) 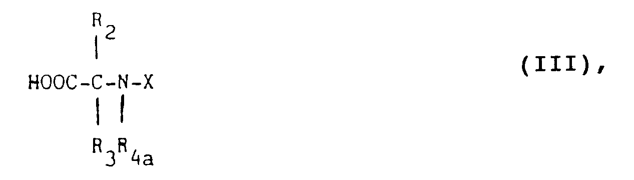 worin R₂ und R₃ wie in Anspruch 1 definiert sind, R₄ₐ Wasserstoff, C₁-C₆-Alkyl oder Cyclopropyl darstellt, und X eine Urethan-Schutzgruppe bedeutet, gefolgt vom Entfernen der Schutzgruppe X, oder
b) Herstellen einer Verbindung der allgemeinen Formel (I), worin R₄ Wasserstoff, C₁-C₆-Alkyl oder Cyclopropyl darstellt, durch Umsetzen einer Verbindung der allgemeinen Formel (IV) 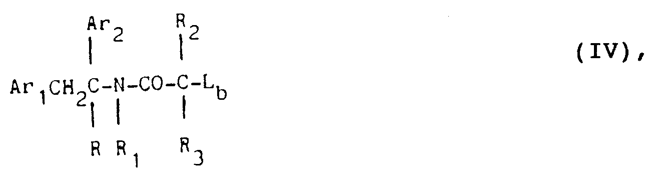 worin R, R₁, R₂, R₃, Ar₁ und Ar₂ wie in Anspruch 1 definiert sind, und L_{b} eine Abgangsgruppe darstellt, mit einem Amin der allgemeinen Formel (V)
H₂NR_{4b} (V),
worin R_{4b} Wasserstoff, C₁-C₆-Alkyl oder Cyclopropyl bedeutet, oder
c) Herstellen einer Verbindung der allgemeinen Formel (I), worin R₄ eine Gruppe -COCHR₅NH₂ darstellt, durch Umsetzen eines Amins der allgemeinen Formel (VI) 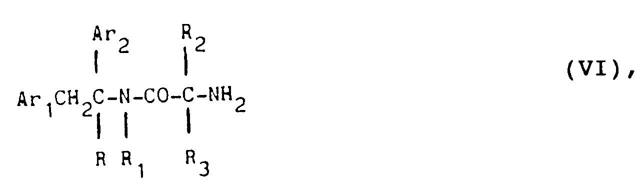 worin R, R₁, R₂, R₃, Ar₁ und Ar₂ wie in Anspruch 1 definiert sind, mit einer Verbindung der allgemeinen Formel VII
HO(C=O)CHR₅NHX (VII),
worin R₅ wie in Anspruch 1 definiert ist, und X wie oben definiert ist, gefolgt vom Entfernen der Schutzgruppe X, und,
wenn notwendig oder erwünscht, Überführen der so erhaltenen Verbindung der Formel (I) in ein pharmazeutisch annehmbares Salz hievon oder umgekehrt,
umfaßt.

## Patentansprüche (Patentansprüche für folgende(n) Vertragsstaat(en): ES, GR)

1. Verfahren zur Herstellung einer Verbindung der allgemeinen Formel (I) 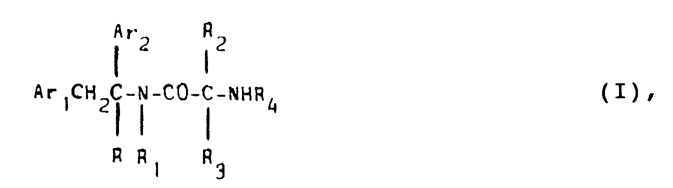 worin Ar₁ und Ar₂, die gleich oder verschieden sein können, gegebenenfalls durch Fluor substituiertes Phenyl darstellen, R₁ und R₂, die gleich oder verschieden sein können, Wasserstoff oder C₁-C₄-Alkyl bedeuten, R₃ Wasserstoff, C₁-C₄-Alkyl, Phenylmethyl oder 2-(Aminocarbonyl)-ethyl ist, und R₄ Wasserstoff, C₁-C₆-Alkyl, Cyclopropyl oder eine Gruppe -COCH(R₅)NH₂ darstellt, worin R₅ Wasserstoff oder Methyl bedeutet, R C₁-C₄-Alkyl ist, vorausgesetzt, daß, wenn R₂ und R₃ beide Wasserstoff darstellen, R₄ von Wasserstoff verschieden ist; oder eines pharmazeutisch annehmbaren Salzes hievon, welches Verfahren
a) das Herstellen einer Verbindung der allgemeinen Formel (I), worin R₄ Wasserstoff, C₁-C₆-Alkyl oder Cyclopropyl darstellt, durch direktes Koppeln eines Amins der allgemeinen Formel (II) 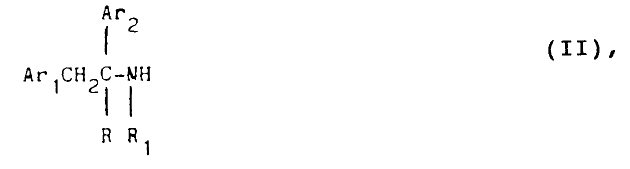 worin R, R₁, Ar₁ und Ar₂ wie oben definiert sind, mit einer Verbindung der allgemeinen Formel (III) 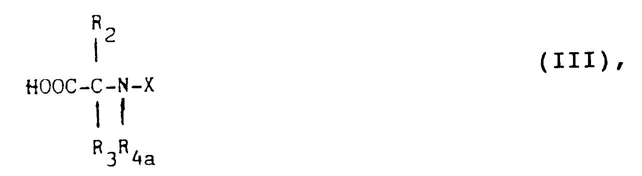 worin R₂ und R₃ wie oben definiert sind, R₄ₐ Wasserstoff, C₁-C₆-Alkyl oder Cyclopropyl darstellt, und X eine Urethan-Schutzgruppe bedeutet, gefolgt vom Entfernen der Schutzgruppe X, oder
b) Herstellen einer Verbindung der allgemeinen Formel (I), worin R₄ Wasserstoff, C₁-C₆-Alkyl oder Cyclopropyl darstellt, durch Umsetzen einer Verbindung der allgemeinen Formel (IV) 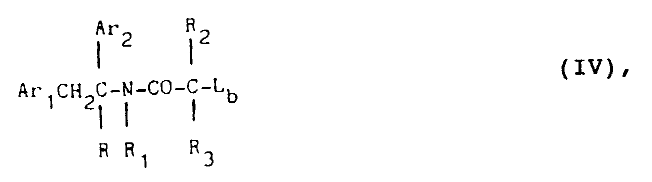 worin R, R₁, R₂, R₃, Ar₁ und Ar₂ wie oben definiert sind, und L_{b} eine Abgangsgruppe darstellt, mit einem Amin der allgemeinen Formel (V)
H₂NR_{4b} (V),
worin R_{4b} Wasserstoff, C₁-C₆-Alkyl oder Cyclopropyl bedeutet, oder
c) Herstellen einer Verbindung der allgemeinen Formel (I), worin R₄ eine Gruppe -COCHR₅NH₂ darstellt, durch Umsetzen eines Amins der allgemeinen Formel (VI) 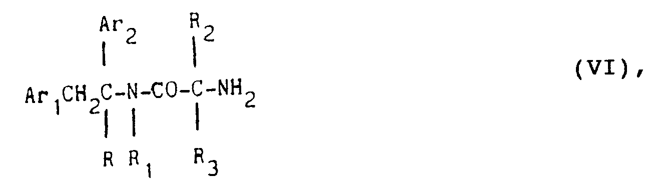 worin R, R₁, R₂, R₃, Ar₁ und Ar₂ wie oben definiert sind, mit einer Verbindung der allgemeinen Formel VII
HO(C=O)CHR₅NHX (VII),
worin R₅ und X wie oben definiert sind, gefolgt vom Entfernen der Schutzgruppe X, und,
wenn notwendig oder erwünscht, Überführen der so erhaltenen Verbindung der Formel (I) in ein pharmazeutisch annehmbares Salz hievon oder umgekehrt,
umfaßt.

2. Verfahren nach Anspruch 1, worin R₂ Wasserstoff ist.

3. Verfahren nach Anspruch 1, worin R₁ und R₂ aus Wasserstoff und Methyl ausgewählt sind, R Methyl darstellt, R₃ Wasserstoff ist und R₄ die Bedeutung -COCHR₅NH₂ hat.

4. Verfahren nach Anspruch 1 oder 2, worin Ar₁ und Ar₂ aus Phenyl und 4-Fluorphenyl ausgewählt sind, R₁ Wasserstoff oder Methyl darstellt, R₃ C₁-C₄-Alkyl, Phenylmethyl oder 2-(Aminocarbonyl)-ethyl bedeutet und R₄ Wasserstoff ist.

5. Verfahren nach Anspruch 1, worin Ar₁ und Ar₂ aus Phenyl und 4-Fluorphenyl ausgewählt sind, R₁ und R₂ Wasserstoff oder Methyl darstellen, R₃ Wasserstoff bedeutet und R₄ C₁-C₄-Alkyl oder Cyclopropyl ist.

6. Verfahren nach Anspruch 1, bei welchem die Verbindung der allgemeinen Formel (I)
2-Glycinamido-N-(1,2-diphenyl-1-methylethyl)-acetamidhydrochlorid;
(2S)-Glycinamido-N-(1,2-diphenyl-1-methylethyl)-propanamid;
2-(L-Alaninamido)-N-(1,2-diphenyl-1-methylethyl)-acetamid;
(2S)-2-(L-Alaninamido)-N-(1,2-diphenyl-1-methylethyl)-propanamid;
2-Glycinamido-N-[1,2-bis-(4-fluorphenyl)-1-methylethyl]-acetamid;
(2S)-2-Amino-3-phenyl-N-(1,2-diphenyl-1-methylethyl)-propanamidhydrochlorid;
(2R)-2-Amino-3-phenyl-N-(1,2-diphenyl-1-methylethyl)-propanamidhydrochlorid;
(2S)-2-Amino-N-(1,2-diphenyl-1-methylethyl)-propanamidhydrochlorid;
(2R)-2-Amino-N-(1,2-diphenyl-1-methylethyl)-propanamidhydrochlorid;
2-Amino-N-(1,2-diphenyl-1-methylethyl)-2-methylpropanamid;
(2S)-2-Amino-4-(aminocarbonyl)-N-(1,2-diphenyl-1-methyl-ethyl)-butanamidmaleat;
(2R)-2-Amino-4-(aminocarbonyl)-N-(1,2-diphenyl-1-methyl-ethyl)-butanamidmaleat;
(2S)-2-Amino-N-[1,2-bis-(4-fluorphenyl)-1-methylethyl]-propanamid;
2-(Methylamino)-N-(1,2-diphenyl-1-methylethyl)-propanamidhydrochlorid;
2-(Methylamino)-N-(1,2-diphenyl-1-methylethyl)-acetamidhydrochlorid;
2-(Ethylamino)-N-(1,2-diphenyl-1-methylethyl)-acetamidhydrochlorid;
2-(Propylamino)-N-(1,2-diphenyl-1-methylethyl)-acetamidhydrochlorid;
2-(Isopropylamino)-N-(1,2-diphenyl-1-methylethyl)-acetamidhydrochlorid;
2-(Cyclopropylamino)-N-(1,2-diphenyl-1-methylethyl)-acetamidhydrochlorid;
2-(Butylamino)-N-(1,2-diphenyl-1-methylethyl)-acetamidhydrochlorid;
2-(Hexylamino)-N-(1,2-diphenyl-1-methylethyl)-acetamidhydrochlorid;
2-(Methylamino)-N-(1,2-diphenyl-1-methylethyl)-acetamidhydrochlorid;
(+)-2-(Methylamino)-N-(1,2-diphenyl-1-methylethyl)-acetamidhydrochlorid;
(-)-2-(Methylamino)-N-(1,2-diphenyl-1-methylethyl)-acetamid;
2-(Methylamino)-N-[1,2-bis-(4-flourphenyl)-1-methylethyl]-acetamidhydrochlorid;
2-(Butylamino)-N-[1,2-bis-(4-fluorphenyl)-1-methylethyl]-acetamidhydrochlorid;
2-(Methylamino)-N-(1-ethyl-1,2-diphenylethyl)-acetamidhydrochlorid;
2-(Butylamino)-N-(1-ethyl-1,2-diphenylethyl)-acetamidhydrochlorid;
oder ein pharmazeutisch annehmbares Salz von einem hievon ist.

## Revendications (Revendications pour l'(les) Etat(s) contractant(s) suivant(s): AT, BE, CH, DE, FR, GB, IT, LI, LU, NL, SE)

1. Composé de formule générale I. 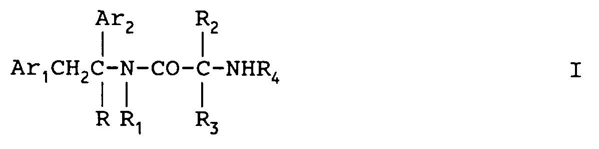 où
Ar₁ et Ar₂, qui peuvent être identiques ou différents, représentent phényle facultativement substitué par fluor,
R₁ et R₂, qui peuvent être identiques ou différents, représentent hydrogène ou alcoyle en C₁₋₄,
R₃ représente hydrogène, alcoyle en C₁₋₄, phénylméthyle ou 2-(aminocarbonyl)éthyle, et
R₄ représente hydrogène, alcoyle en C₁₋₆, cyclopropyle, ou un radical -COCH(R₅)NH₂ où R₅ représente hydrogène ou méthyle,
R représente alcoyle en C₁₋₄,
avec la restriction que lorsque R₂ et R₃ représentent tous deux hydrogène, alors R₄ est autre qu'hydrogène,
ou un sel pharmaceutiquement acceptable de celui-ci.

2. Composé suivant la revendication 1, dans lequel R₂ est hydrogène.

3. Composé suivant la revendication 1, dans lequel R₁ et R₂ sont choisis parmi hydrogène et méthyle, R représente méthyle, R₃ représente hydrogène et R₄ représente -COCHR₅NH₂.

4. Composé suivant la revendication 1 ou 2, dans lequel Ar₁ et Ar₂ sont choisis parmi phényle et 4-fluorophényle, R₁ représente hydrogène ou méthyle, R₃ représente alcoyle en C₁₋₄, phénylméthyle ou 2-(aminocarbonyl)éthyle, et R₄ représente hydrogène.

5. Composé suivant la revendication 1, dans lequel Ar₁ et Ar₂ sont choisis parmi phényle et 4-fluorophényle, R₁ et R₂ représentent hydrogène ou méthyle, R₃ représente hydrogène et R₄ représente alcoyle en C₁₋₄ ou cyclopropyle.

6. Composé suivant la revendication 1, qui est :
le chlorhydrate de 2-glycinamido-N-(1,2-diphényl-1-méthyléthyl)acétamide;
le (2S)-2-glycinamido-N-(1,2-diphényl-1-méthyléthyl)-propanamide;
le 2-(L-alaninamido)-N-(1,2-diphényl-1-méthyléthyl)-acétamide;
le (2S)-2-(L-alaninamido)-N-(1,2-diphényl-1-méthyléthyl)propanamide;
le 2-glycinamido-N-[1,2-bis(4-fluorophényl)-1-méthyléthyl]acétamide;
le chlorhydrate de (2S)-2-amino-3-phényl-N-(1,2-diphényl-1-méthyléthyl)propanamide;
le chlorhydrate de(2R)-2-amino-3-phényl-N-(1,2-diphényl-1-méthyléthyl)propanamide;
le chlorhydrate de (2S)-2-amino-N-(1,2-diphényl-1-méthyléthyl)propanamide;
le chlorhydrate de (2R)-2-amino-N-(1,2-diphényl-1-méthyléthyl)propanamide;
le 2-amino-N-(1,2-diphényl-1-méthyléthyl)-2-méthylpropanamide;
le maléate de (2S)-2-amino-4-(aminocarbonyl)-N-(1,2-diphényl-1-méthyléthyl)butanamide;
le maléate de (2R)-2-amino-4-(aminocarbonyl)-N-(1,2-diphényl-1-méthyléthyl)butanamide;
le (2S)-2-amino-N-[1,2-bis(4-fluorophényl)-1-méthyléthyl]- propanamide;
le chlorhydrate de 2-(méthylamino)-N-(1,2-diphényl-1-méthyléthyl)propanamide;
le chlorhydrate de 2-(méthylamino)-N-(1,2-diphényl-1-méthyléthyl)acétamide;
le chlorhydrate de 2-(éthylamino)-N-(1,2-diphényl-1-méthyléthyl)acétamide;
le chlorhydrate de 2-(propylamino)-N-(1,2-diphényl-1-méthyléthyl)acétamide;
le chlorhydrate de 2-(isopropylamino-N-(1,2-diphényl-1-méthyléthyl)acétamide;
le chlorhydrate de 2-(cyclopropylamino)-N-(1,2-diphényl-1-methyléthyl)acétamide;
le chlorhydrate de 2-(butylamino)-N-(1,2-diphényl-1-méthyléthyl)acétamide;
le chlorhydrate de 2-(hexylamino)-N-(1,2-diphényl-1-méthyléthyl)acétamide;
le chlorhydrate de 2-(méthylamino)-N-(1,2-diphényl-1-méthyléthyl)acétamide;
le chlorhydrate de (+)-2-(méthylamino)-N-(1,2-diphényl-1-méthyléthyl)acétamide;
le (-)-2-(méthylamino)-N-(1,2-diphényl-1-méthyléthyl)acétamide;
le chlorhydrate de 2-(méthylamino)-N-[1,2-bis(4-fluorophényl)-1-méthyléthyl]acétamide;
le chlorhydrate de 2-(butylamino)-N-[1,2-bis(4-fluorophényl)-1-méthyléthyl]acétamide;
le chlorhydrate de 2-(méthylamino)-N-(1-éthyl-1,2-diphényléthyl)acétamide;
le chlorhydrate de 2-(butylamino)-N-(1-éthyl-1,2-diphényléthyl)acétamide;
ou un sel d'addition d'acide pharmaceutiquement acceptable de l'un quelconque de ceux-ci.

7. Utilisation d'un composé de formule I tel que défini dans la revendication 1 dans la fabrication d'un médicament pour le traitement de l'épilepsie.

8. Composition pharmaceutique comprenant moins de 80% en poids d'un composé de formule générale I ou d'un sel d'addition d'acide pharmaceutiquement acceptable de celui-ci, en mélange avec un adjuvant, diluant ou excipient pharmaceutiquement acceptable.

9. Procédé de préparation d'un composé de formule générale I tel que défini dans la revendication 1, lequel procédé comprend :
a) la production d'un composé de formule générale I où R₄ représente hydrogène, alcoyle en C₁₋₆ ou cyclopropyle, par condensation directe d'une amine de formule générale II : 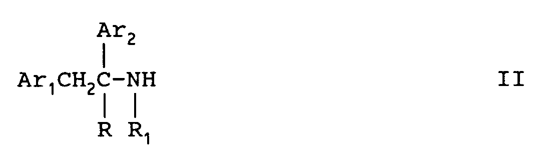 où R, R₁, Ar₁ et Ar₂ sont tels que définis dans la revendication 1, avec un composé de formule générale III : 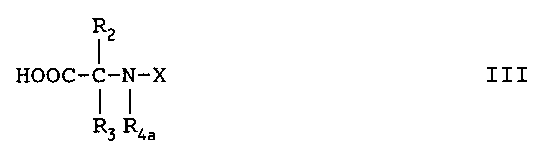 où R₂ et R₃ sont tels que définis dans la revendication 1, R₄ₐ représente hydrogène, alcoyle en C₁₋₆ ou cyclopropyle et X représente un radical protecteur d'uréthanne suivie par l'élimination du radical protecteur X, ou
b) la production d'un composé de formule générale I, où R₄ représente hydrogène, alcoyle C₁₋₆ ou cyclopropyle, par réaction d'un composé de formule générale IV : 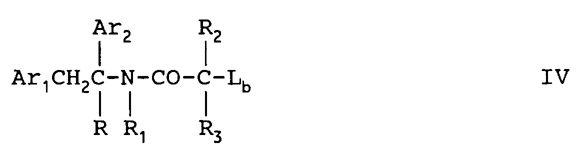 où R, R₁, R₂, R₃, Ar₁ et Ar₂ sont tels que définis dans la revendication 1 et L_{b} représente un radical partant, avec une amine de formule générale V :
H₂NR_{4b}
où R_{4b} représente hydrogène, alcoyle en C₁₋₆ ou cyclopropyle, ou
c) la production d'un composé de formule générale I, où R₄ représente un radical -COCHR₅NH₂, par réaction d'une amine de formule générale VI : 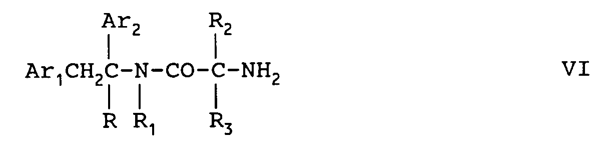 où R, R₁, R₂, R₃, Ar₁ et Ar₂ sont tels que définis dans la revendication 1, avec un composé de formule générale VII :
HO(C=O)CHR₅NHX VII
où R₅ est tel que défini dans la revendication 1 et X est tel que défini ci-dessus, suivie de l'élimination du radical protecteur X,
et, si nécessaire ou souhaité, la conversion du composé de formule générale I ainsi obtenu en un sel pharmaceutiquement acceptable de celui-ci ou vice versa.

## Revendications (Revendications pour l'(les) Etat(s) contractant(s) suivant(s): ES, GR)

1. Procédé pour préparer un composé de formule générale I : 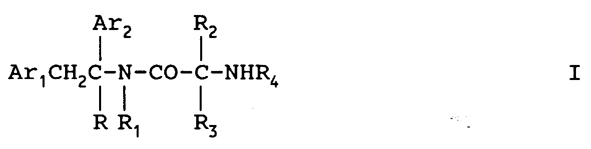 où
Ar₁ et Ar₂, qui peuvent être identiques ou différents, représentent phényle facultativement substitué par-fluor,
Ri et R₂, qui peuvent être identiques ou différents, représentent hydrogène ou alcoyle en C₁₋₄,
R₃ représente hydrogène, alcoyle en C₁₋₄, phénylméthyle ou 2-(aminocarbonyl)éthyle, et
R₄ représente hydrogène, alcoyle en C₁₋₆, cyclopropyle, ou un radical -COCH(R₅)NH₂ où R₅ représente hydrogène ou méthyle,
R représente alcoyle en C₁₋₄,
avec la restriction que lorsque R₂ et R₃ représentent tous deux hydrogène, alors R₄ est autre qu'hydrogène,
ou un sel d'addition d'acide pharmaceutiquement acceptable de celui-ci,
lequel procédé comprend :
a) la production d'un composé de formule générale I, où R₄ représente hydrogène, alcoyle en C₁₋₆ ou cyclopropyle, par condensation directe d'une amine de formule générale II : 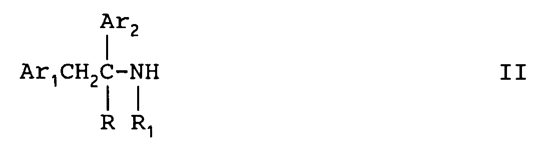 où R, R₁, Ar₁ et Ar₂ sont tels que définis ci-dessus, avec un composé de formule générale III : 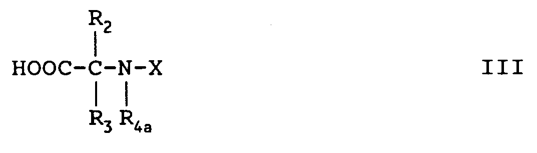 où R₂ et R₃ sont tels que définis ci-dessus, R₄ₐ représente hydrogène, alcoyle en C₁₋₆ ou cyclopropyle et X représente un radical protecteur d'uréthanne, suivie de l'élimination du radical protecteur X, ou
b) la production d'un composé de formule générale I, où R₄ représente hydrogène, alcoyle C₁₋₆ ou cyclopropyle, par réaction d'un composé de formule générale IV : 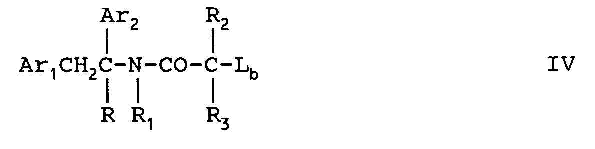 où R, R₁, R₂, R₃, Ar₁ et Ar₂ sont tels que définis ci-dessus, et L_{b} représente un radical partant avec une amine de formule générale V :
H₂NR_{4b} V
où R_{4b} représente hydrogène, alcoyle en C₁₋₆ ou cyclopropyle, ou
c) la production d'un composé de formule générale I, où R₄ représente un radical -COCHR₅NH₂, par réaction d'une amine de formule générale VI : 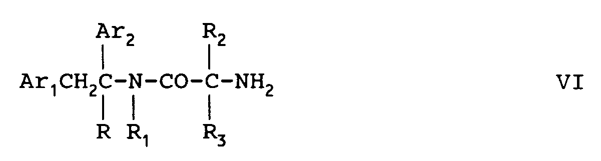 où R, R₁, R₂, R₃, Ar₁ et Ar₂ sont tels que définis ci-dessus, avec un composé de formule générale VII :
HO(C=O)CHR₅NHX VII
où R₅ et X sont tels que définis ci-dessus, suivie de l'élimination du radical protecteur X,
et, si nécessaire ou souhaité, la conversion du composé de formule générale I ainsi obtenu en un sel pharmaceutiquement acceptable de celui-ci ou vice versa.

2. Procédé suivant la revendication 1, dans lequel R₂ est hydrogène.

3. Procédé suivant la revendication 1, dans lequel R₁ et R₂ sont choisis parmi hydrogène et méthyle, R représente méthyle, R₃ représente hydrogène et R₄ représente -COCHR₅NH₂.

4. Procédé suivant la revendication 1 ou 2, dans lequel Ar₁ et Ar₂ sont choisis parmi phényle et 4-fluorophényle, R₁ représente hydrogène ou méthyle, R₃ représente alcoyle en C₁₋₄, phénylméthyle ou 2-(aminocarbonyl)éthyle et R₄ représente hydrogène.

5. Procédé suivant la revendication 1, dans lequel Ar₁ et Ar₂ sont choisis parmi phényle, et 4-fluorophényle, R₁ et R₂ représentent hydrogène ou méthyle, R₃ représente hydrogène et R₄ représente alcoyle en C₁₋₄ ou cyclopropyle.

6. Procédé suivant la revendication 1, dans lequel le composé de formule générale I est :
le chlorhydrate de 2-glycinamido-N-(1,2-diphényl-1-méthyléthyl) acétamide;
le (2S)-2-glycinamido-N-(1,2-diphényl-1-méthyléthyl)propanamide;
le 2-(L-alaninamido)-N-(1,2-diphényl-1-méthyléthyl)acétamide;
le (2S)-2-(L-alaninamido)-N-(1,2-diphényl-1-méthyléthyl)propanamide;
le 2-glycinamido-N-[1,2-bis(4-fluorophényl)-1-méthyléthyl]acétamide;
le chlorhydrate de (2S)-2-amino-3-phényl-N-(1,2-diphényl-1-méthyléthyl)propanamide;
le chlorhydrate de (2R)-2-amino-3-phényl-N-(1,2-diphényl-1-méthyléthyl)propanamide;
le chlorhydrate de (2S)-2-amino-N-(1,2-diphényl-1-méthyléthyl)propanamide;
le chlorhydrate de (2R)-2-amino-N-(1,2-diphényl-1-méthyléthyl)propanamide;
le 2-amino-N-(1,2-diphényl-1-méthyléthyl)-2-méthylpropanamide;
le maléate de (2S)-2-amino-4(aminocarbonyl)-N-(1,2-diphényl-1-méthyléthyl)butanamide;
le maléate de (2R)-2-amino-4-(aminocarbonyl)-N-(1,2-diphényl-1-méthyléthyl)butanamide;
le (2S)-2-amino-N-[1,2-bis(4-fluorophényl)-1-méthyléthyl]propanamide;
le chlorhydrate de 2-(méthylamino)-N-(1,2-diphényl-1-méthyléthyl)propanamide;
le chlorhydrate de 2-(méthylamino)-N-(1,2-diphényl-1-méthyléthyl)acétamide;
le chlorhydrate de 2-(éthylamino)-N-(1,2-diphényl-1-méthyléthyl)acétamide;
le chlorhydrate de 2-(propylamino)-N-(1,2-diphényl-1-méthyléthyl)acétamide;
le chlorhydrate de 2-(isopropylamino-N-(1,2-diphényl-1-méthyléthyl)acétamide;
le chlorhydrate de 2-(cyclopropylamino)-N-(1,2-diphényl-1-méthyléthyl)acétamide;
le chlorhydrate de 2-(butylamino)-N-(1,2-diphényl-1-méthyléthyl)acétamide;
le chlorhydrate de 2-(hexylamino)-N-(1,2-diphényl-1-méthyléthyl)acétamide;
le chlorhydrate de 2-(méthylamino)-N-(1,2-diphényl-1-méthyléthyl)acétamide;
le chlorhydrate de (+)-2-(méthylamino)-N-(1,2-diphényl-1-méthyléthyl)acétamide;
le (-)-2-(méthylamino)-N-(1,2-diphényl-1-méthyléthyl)acétamide;
le chlorhydrate de 2-(méthylamino)-N-[1,2-bis(4-fluorophényl)-1-méthyléthyl]acétamide;
le chlorhydrate de 2-(butylamino)-N-[1,2-bis(4-fluorophényl)-1-méthyléthyl]acétamide;
le chlorhydrate de 2-(méthylamino)-N-(1-éthyl-1,2-diphényléthyl)acétamide;
le chlorhydrate de 2-(butylamino)-N-(1-éthyl-1,2-diphényléthyl)acétamide;
ou un sel d'addition d'acide pharmaceutiquement acceptable de l'un quelconque de ceux-ci.
